# EUROPEAN PATENT APPLICATION

(11) **EP 2 893 953 A1**
(43) Date of publication of application: **15.07.2015**
(21) Application number: 13835163.0
(22) Date of filing: 04.09.2013
(51) Int. Cl.: A61N 1/05

(54) **INTRACAVITARY ELEMENT**

(30) Priority: 06.09.2012 ES 201231383
(71) Applicant: Sanchez Jaime, Maria del Pilar, 08970 Barcelona (ES)
(72) Inventor: Sanchez Jaime, Maria del Pilar, 08970 Barcelona (ES)
(74) Representative: Diaz Nunez, Joaquin
(86) International application number: PCT/ES2013/000196
(87) International publication number: WO 2014/037591

(57) **Abstract**

The invention relates to an intracavitary element based on a long, substantially cylindrical body that is joined, at the proximal end thereof, to the cable (2) that connects it to the apparatus, and comprising a grip (3), in the proximal region, that is thicker than the rest of the body and has a flat area (10) on the upper part thereof; a straight and cylindrical central region (4); and a distal region (5) that has a bulge (7) on the upper face and is bent in relation to the axial axis of the central region (4), at an angle (a) oscillating between 1 and 90°. The element has one, two or more independent electrodes (6) distributed between the central region (4) and the distal region (5), which are separated by means of pieces (8) of insulating material when they are more than one, and have temperature sensors (9).

## Description

### Object of the invention

The invention, as stated the title of the present specification, relates to an intracavitary element, which has innovative characteristics and advantages, which will be described in more detail later and that represent a remarkable improvement in the current state of the art.

More particularly, the object of the invention is focused on an intracavitary element of the type consisting of an accessory device designed for use with a molecular bio-resonance equipment, of the type that generate the passage of alternating current conducted via electrodes, to the treated area, said element being specifically applicable for intracavitary therapies, that is, that are carried out within a body cavity, and having a series of improvements, relating to both its structural configuration and to the constitution and arrangement of the incorporated electrodes, that improve significantly the performance, versatility and effectiveness thereof in relation to the similar accessory elements currently existing in the market for the same purpose.

### Field of application

The field of application of the present invention falls within the sector of industry dedicated to the manufacture of equipment, apparatuses and devices for medical therapeutic and aesthetic treatments, focusing particularly on those that apply electric current.

### Background of the invention

As it is known, by means of the type of apparatuses that here concerns, a beam of current is directed to the site of application, this being absorbed by the tissues, which serves as a therapy for various medical and aesthetic treatments. To that end, the current is applied through electrodes that may be from different characteristics, which are incorporated in a supporting accessory element, which is that comes into contact with the body area to be treated, being connected to a generator equipment that is responsible for generating the current at certain frequencies and powers and with specific waveforms.

Among the different accessory elements that can be coupled to these equipments, are the intracavitary ones that, as outlined previously, are specially designed for therapies carried out within a body cavity, as it can be the vagina or rectum.

As a reference to the current state of the art, it should be noted that it is aware the existence in the market of various accessory elements of the type that here concerns, However, none of them has some technical, structural, and constitutive characteristics similar to the ones presented by the intracavitary element here proposed, as claimed.

### Explanation of the invention

Concretely, that proposed by the present invention consists of an intracavitary element especially applicable for use with molecular bio-resonance therapy equipment, which is specially developed for intracavitary treatments in Gynecology and Urology and which, in a characterizing way, brings together a series of innovative characteristics, in its structural configuration and in the arrangement of the electrodes, which optimize the treatment.

Thus, both the shape and dimensions of the active part are carefully studied to optimize the results. In particular, it has a straight central body and an angled end portion. This end portion, moreover, is asymmetrically shaped in order to facilitate the access to the sites of treatment.

In addition, it can incorporate various models of electrodes of different size, especially at the end portion, to be adapted to different types of treatment.

While the body of the intracavitary element incorporates at least one electrode and may incorporate up to three or four independent electrodes to supply radio frequency energy to the treated area, in a preferred embodiment of the invention, it has two independent electrodes. In such preferred option, an electrode is located on the middle region and other in the distal region. The power delivered by these electrodes is controlled independently.

The electrodes that the accessory element incorporates have temperature sensors, to monitor this parameter in real-time, allowing incorporating security and treatment automation measures.

Furthermore, the electrodes of the intracavitary element are preferably isolated active electrodes (capacitive), but without involving a limitation since they may also be non-isolated metal electrodes (resistive).

Finally, the grip of the intracavitary element proposed by the invention, with which holds and handles, has a specially designed shape which, advantageously, allows the operator to know at all times which is the axis of the device. This is because, in the application, the active portion of the element to be inserted in the corresponding body cavity is not visible, and the operator needs to know the position of the end portion of the accessory.

In the light of the above, it is found that the described intracavitary element represents an innovative structure of structural and constitutive characteristics unknown so far to this end, reasons which in combination with its practical utility, provide it with enough basis to obtain the exclusivity privilege which is applied for.

### Description of the drawings

In order to complement the description that is being carried out and with the object to help to a better understanding of the invention, a set of drawings is accompanied to the present specification as an integral part thereof, in which, with an illustrative and non-limiting character, the following has been represented:

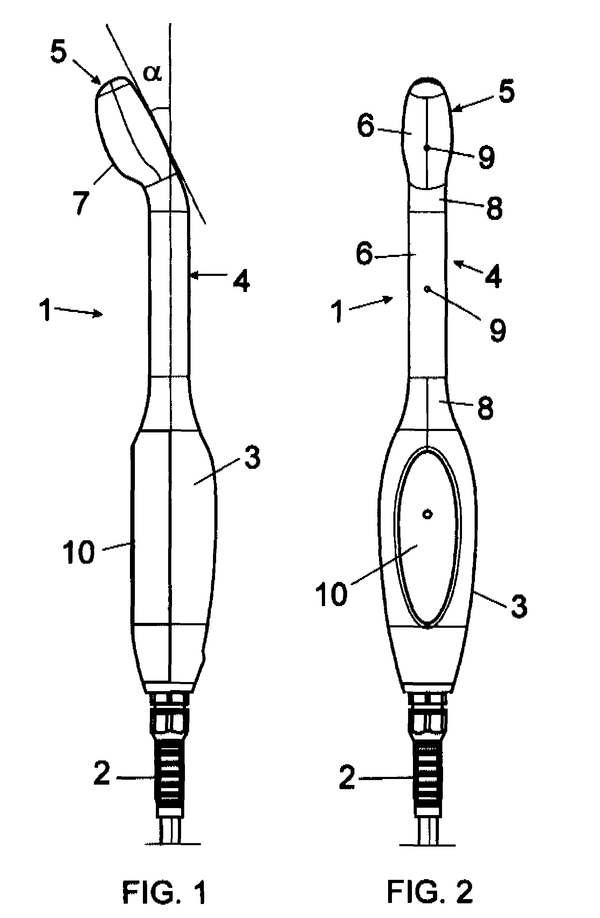

Figures 1 and 2. They show respective side elevation and top views, respectively, of an exemplary embodiment of the intracavitary element object of the invention, which show their general external configuration as well as the main comprising parts and elements.

### Preferred embodiment of the invention

In light of the previously mentioned figures, and according to the numeration adopted, an example of a preferred and non-limiting embodiment of the intracavitary element can be observed therein, which comprises the parts and elements which are indicated and described in more detail below.

Thus, as seen in said Figures, the involved accessory (1) is configured from a long, substantially cylindrical body that, joined, at the proximal end thereof, to the cable (2) that connects it to the current generator apparatus, comprises a grip (3), located on the proximal region, the closest to said cable (2), and that is markedly thicker than the rest of the body; a totally straight and cylindrical central region (4), intended to facilitate the penetration of the accessory in the body cavity and reach the site to be treated; and a distal region (5), which is substantially bent in relation to the axial axis of the central region (4), said bending having an angle (a) which can range between 1 and 90°, while it is preferably located at 30°.

Moreover, the distal region (5) has a bulge (7) located on the upper face thereof, towards which the described bending is tilted, intended to facilitate the access to the sites of treatment.

While the distal region (5) is the active region where the electrodes (6) are preferably located to supply radio frequency energy to the treated region, these, since they may be one, two, or more, are distributed between the straight central region (4) and the distal region (5).

Said electrodes (6), in the case of two or more, are independent, for which are separated by means of pieces (8) of insulating material.

In the example depicted in the Figures, the element has two electrodes (6), one is located in the central region (3) and the other in the distal region (5) and, being independent, the power delivered by them is controlled independently.

In addition, each of said electrodes (6) has temperature sensor (9).

It should be noted, finally, that in the grip (3) is provided a flat area (10) on the upper part thereof, towards which the distal region (5) is bent and is intended to allow the operator to notice which is the position of the distal region (5) of the device, although it can not see it.

Having sufficiently described the nature of the present invention, it is not considered necessary to further extend its explanation for any person skilled in the art to understand its scope and the advantages derived therefrom, stating that, within its essence, it can be put into practice in other embodiments provided that its fundamental principle is not altered, changed or modified.

## Claims

1. INTRACAVITARY ELEMENT, of the type applicable for Gynecology and Urology therapies, and that is used with a molecular bio-resonance equipment carried out by electrodes (6) incorporated therein, and is configured from a long, substantially cylindrical body that is joined, at the proximal end thereof, to the cable (2) that connects it to the current generator apparatus, **characterized in that** said body comprises a grip (3), in the proximal region, that is markedly thicker than the rest of the body; a totally straight and cylindrical central region (4); and a distal region (5), which is substantially bent in relation to the axial axis of the central region (4); and **in that** it has one, two or more independent electrodes (6), distributed between the central region (4) and the distal region (5), being separated by means of pieces (8) of insulating material when they are more than one.

2. INTRACAVITARY ELEMENT, according to claim 1, **characterized in that** the bending of the distal region (5) in relation to the axial axis of the central region (4) has an angle (a) which can range between 1 and 90°.

3. INTRACAVITARY ELEMENT, according to claim 1 or 2, **characterized in that** the distal region (5) has a bulge (7) located on the upper face thereof, towards which the bending thereof is tilted.

4. INTRACAVITARY ELEMENT, according to claims 1-3, **characterized in that** the electrodes (6) have temperature sensors (9).

5. INTRACAVITARY ELEMENT, according to claims 1-4, **characterized in that** the grip (3) has a flat area (10) on the upper part thereof, towards which the distal region (5) is bent.

## Amended claims

### Amended claims under Art. 19.1 PCT

**1.** INTRACAVITARY ELEMENT, of the type applicable for Gynecology and Urology therapies, and that is used with a molecular bio-resonance equipment carried out by electrodes (6) incorporated therein, and is configured from a long, substantially cylindrical body that is joined, at the proximal end thereof, to the cable (2) that connects it to the current generator apparatus, comprising a grip (3), in the proximal region, that is markedly thicker than the rest of the body, a straight and cylindrical central region (4), and a distal region (5), which is substantially bent in relation to the axial axis of the central region (4), is **characterized in that** it has two or more independent electrodes (6), that is, that the power delivered by the same is controlled independently, and **in that** also distributed between the central region (4) and the distal region (5), said electrodes (6) being separated from each other by means of pieces (8) of insulating material.

**2.** INTRACAVITARY ELEMENT, according to claim 1, **characterized in that** la distal region (5) has a bulge (7) located on the upper face thereof, towards which the bending thereof is tilted, intended to facilitate the access to treatment site by means of the electrodes (6).

**3.** INTRACAVITARY ELEMENT, according to claims 1-2, **characterized in that** the grip (3) has a flat area (10) on the upper part thereof, towards which the distal region (5) is bent.
